**Europäisches Patentamt**

**European Patent Office**

**Office européen des brevets**

(11) Veröffentlichungsnummer : **0 069 228**
**B1**

(12) **EUROPÄISCHE PATENTSCHRIFT**

(45) Veröffentlichungstag der Patentschrift :
10.09.86

(21) Anmeldenummer : 82104541.6

(22) Anmeldetag : 25.05.82

(51) Int. Cl.⁴ : **A 61 L   2/20, B 65 B 55/00**

(54) **Verfahren und Vorrichtung zum Sterilisieren von becherförmigen Behältern.**

(30) Priorität : 27.06.81 DE 3125430

(43) Veröffentlichungstag der Anmeldung :
12.01.83 Patentblatt 83/02

(45) Bekanntmachung des Hinweises auf die Patenterteilung : 10.09.86 Patentblatt 86/37

(84) Benannte Vertragsstaaten :
CH DE FR GB IT LI SE

(56) Entgegenhaltungen :
WO-A-79 /010 74
GB-A-  880 404

(73) Patentinhaber : Kolbus GmbH & Co. KG
Osnabrücker Strasse 77
D-4993 Rahden (DE)

(72) Erfinder : Hick, Werner
Eichstrasse 7
D-4901 Hiddenhausen 4 (DE)

**Beschreibung**

Die Erfindung betrifft ein Verfahren zum Sterilisieren von becherförmigen Behältern unter Verwendung eines Sterilisierungsmittels, das sich als Kondensatfilm an der Behälterwand niederschlägt und sie betrifft ferner eine Vorrichtung zur Durchführung des Verfahrens.

Zum Abtöten von Mikroorganismen in Nahrungsmittelbehältern gibt es verschiedene Sterilisierungsverfahren, wie die UV-Bestrahlung, Einblasen eines aufgeheizten Wasserdampf-Luftgemisches und die sogenannte Naß-Aseptik, bei der die Behälterinnenwand mit einem Wasserstoffperoxidgemisch besprüht und durch Heißluft wieder getrocknet wird.

Im Hinblick auf eine möglichst lange Haltbarkeit der Produkte hat sich die Verwendung des Sterilisationsmittels Peroxid als ein sicheres Mikroorganismen abtötendes Mittel erwiesen, das in die Zellhüllen der Mikroorganismen eindringt und zerstörend wirkt.

Trotz Versprühung des Wasserstoffperoxidgemisches läßt sich lediglich eine Benetzung von 35 % der Behälterinnenfläche erzielen. Selbst bei einer noch so feinen Zerstäubung bilden sich auf der Oberfläche Tropfen, zwischen denen unbenetzte Flächen verbleiben.

Da bekanntlich die Behälter nach dem Sterilisieren keine Restmengen an Peroxid aufweisen sollen, ist eine Entfernung dieses toxischen Stoffes in Form von Abdampfen durch Wärmeeinwirkung notwendig. Der Einsatz des Naß-Aseptik-Verfahrens unter Verwendung eines Wasserstoffperoxidgemisches verlangt folglich stets eine Nachbehandlung zum weitestgehenden Entfernen von Restmengen Peroxid.

In der WO-A-79 101 074 wird ein Naß-Aseptik-Verfahren unter Schutz gestellt, bei dem über einen piezoelektrischen Wandler das Stoffgemisch in einer Kammer vernebelt und mittels Druckluft durch ein Leitungssystem zu einer erwärmten, über dem Behälter installierten Düse hintransportiert wird, die die Stoffgemischpartikel in den Behälter sprüht. Um ein Verbinden der Partikel zu Tropfen zu verhindern, ist die Düse in einem mit erwärmter Luft beaufschlagten Raum angeordnet. Mit dem Verfahren und der Vorrichtung gemäß der WO-A-79 101 074 wird zwar eine Verbesserung der Vernebelung des Bakterizids erreicht, jedoch erzielt man aufgrund der relativ langen Wegstrecke bis hin zu den zu sterilisierenden Behältern keine absolut sichere Keimfreimachung seiner gesamten Innenfläche.

Die Aufgabe der Erfindung besteht darin, daß bekannte Naß-Aseptik-Verfahren nebst Vorrichtung derart zu verbessern, daß eine absolut sichere Sterilisierung der Gesamtbehälterinnenfläche erzielt wird und daß eine Nachbehandlung zum Entfernen von Restmengen an Wasserstoffperoxid entfällt.

Die Erfindung macht sich die Erkenntnis zunutze, daß der während der thermischen Zersetzung des Sterilisierungsmittels, wie Wasserstoffperoxidgemisch, freiwerdende Sauerstoff eine besonders stark oxidierende Wirkung im Moment seiner Entstehung entfaltet.

Ausgehend von einem Verfahren zum Sterilisieren von becherförmigen Behältern unter Verwendung eines Sterilisierungsmittels, das sich als Kondensatfilm an der Behälterwand niederschlägt, sieht das Verfahren nach der Erfindung vor, daß ein Wasserstoffperoxidgemisch als Sterilisierungsmittel, durch Aufbringen auf eine Oberfläche von hoher Temperatur im becherförmigen Behälter oder unmittelbar davor explosionsartig zum Verdampfen gebracht wird.

Weitere Ausgestaltungen des Verfahrens und Vorrichtungen zur Durchführung des Verfahrens sind in den Unteransprüchen definiert.

Aus der GB-A-880 404 ist ferner eine transportable Vorrichtung zum Desinfizieren von Räumen bekannt, wobei ein unter Druck stehendes Desinfektionsmittel von einer Streudüse auf ein konisches Heizelement gelangt und hierdurch sich die leicht flüchtigen Bestandteile des Desinfektionsmittels in Form eines Gases, Dampfes oder Nebels im Raum verteilen. Bei diesem Verfahren findet lediglich eine Überführung des Desinfektionsmittels in den gasförmigen Zustand statt. Die für ein sicheres Sterilisieren von becherförmigen Behältern erforderliche thermische Zersetzung des Bakterizids läßt sich diesem Verfahren nicht entnehmen.

Die durch die Erfindung erreichten Vorteile liegen in einer wesentlich verbesserten Sterilisierung der Gesamtbehälterinnenfläche. Beim Aufsprühen des Wasserstoffperoxidgemisches auf eine Oberfläche von hoher Temperatur im becherförmigen Behälter oder unmittelbar davor wird ein Teil des Sterilisationsmittels explosionsartig zum Verdampfen gebracht und schlägt sich auf der Behälterwand feinst verteilt nieder. Ein weiterer Teil des Sterilisationsmittels wird durch die explosionsartige Verdampfung mechanisch in feinste Tröpfchen zerlegt und in voller Konzentration gegen die Behälterinnenwand geschleudert. Dabei spaltet das Sterilisationsmittel unter chemischer Mitwirkung der zu tötenden Mikroben und aufgrund seiner thermischen Energie aus der Heizspirale atomaren Sauerstoff ab, der die Mikroben zerstört. Schließlich zersetzt sich ein weiterer Teil des Sterilisationsmittels thermisch bereits an der hochtemperierten Oberfläche oder auf dem Weg zur Behälterinnenwand und der dabei entstehende atomare Sauerstoff gelangt auf kürzestem Weg und in kürzester Zeit an die Behälterinnenwand, um dort keimabtötend zu wirken, bevor er wieder rekombiniert.

Die mit dem explosionsartigen Verdampfen erzielte Feinstverteilung des Wasserstoffperoxidgemisches ermöglicht die Verwendung von lediglich kleinsten Mengen an Sterilisierungsmitteln, wodurch eine Nachbehandlung zum Entfernen von Restmengen in Fortfall gelangt, zumal auch die Strahlungsenergie der hochtemperierten Oberfläche mithilft, eventuelle Restmengen zu entfernen.

Anhand einer Schemazeichnung sollen nachfolgend Ausführungsbeispiele zur Durchführung des erfindungsgemäßen Verfahrens beschrieben werden.

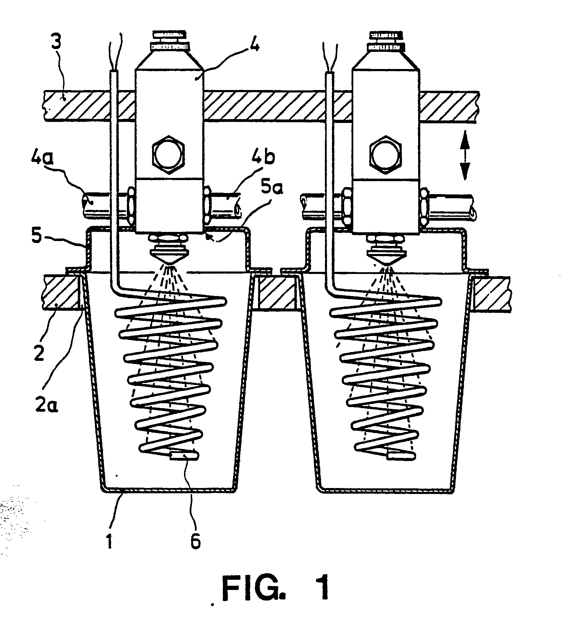

In der Figur 1 wird ein Ausschnitt aus einem bekannten endlosen Becherförderer 2 dargestellt mit mehreren in Reihe liegenden Ausnehmungen 2a zur Aufnahme von beispielsweise mit Molkereiprodukten abzufüllenden Bechern 1. An einem Querträger 3 oberhalb dieses Becherförderers befinden sich ebenfalls in Reihe und mittig über jeder Ausnehmung 2a installiert Sprühdüsen 4 mit einem Anschluß 4a zu einer Druckluftquelle sowie mit einem Anschluß 4b an einen nicht dargestellten Vorratsbehälter mit einem Wasserstoffperoxidgemisch.

Die in die Ausnehmungen 2a eingesetzten Becher 1 werden während des Sterilisiervorgangs durch einen Durchlaß 5a aufweisende Abdeckungen 5 verschlossen, die an den Sprühdüsen 4 befestigt sind, wobei der Düsenaustritt nach unten über die Abdeckung hinausragt.

Jeweils unterhalb der Sprühdüse 4 befindet sich eine kegelstumpfförmig ausgebildete Heizspirale 6 angeordnet, die in den Becher 1 hineinragt und in einem definierten Abstand zum Becherboden endet. Die Heizspirale ist an eine elektrische Spannung gelegt, sie wird ebenso wie die Sprühdüse von dem Querträger 3 getragen und verläuft durch die Abdeckung 5 hindurch.

Gemäß Pfeilrichtung läßt sich der Querträger 3 gemeinsam mit den Sprühdüsen 4, den Abdeckungen 5 und den Heizspiralen 6 taktweise, abgestimmt auf den Vortransport der Becherreihen, zwischen einer unteren und oberen Endstellung verfahren, derart, daß zur Durchführung des Sterilisiervorgangs die Heizspirale 6 in den bereitgestellten Becher kurzzeitig einfährt und der Becher von der Abdeckung 5 verschlossen ist.

In dieser unteren Endstellung werden die Sprühdüsen 4 angesteuert und ein Sprühnebel eines Wasserstoffperoxidgemisches wird in die Becher eingeblasen, der auf die Heizspirale 6 mit einer Oberflächentemperatur von ca. 500 °C trifft und explosionsartig verdampft. Der hierbei entstehende Wasserstoffperoxiddampf schlägt sich als Kondensat an der Becherinnenwand nieder, wodurch eine absolut sichere keimabtötende Wirkung erzielt wird. Vorzugsweise besteht dabei das Wasserstoffperoxidgemisch aus einer 35 %igen Wasserstoffperoxidkonzentration.

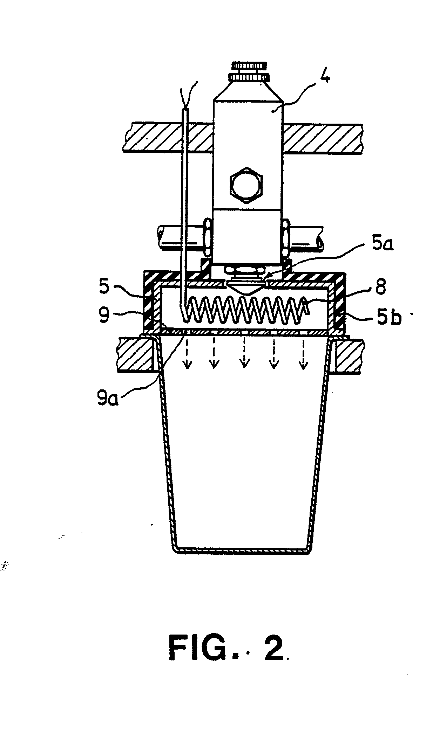

In einem abgewandelten Verfahren kann das Sterilisierungmittel auch unmittelbar vor der Becheröffnung explosionsartig zum Verdampfen gebracht werden. Die Ausgestaltung zur Durchführung dieses Verfahrens sieht, wie Figur 2 veranschaulicht, eine in der Abdeckung 5 installierte Heizspirale 8 vor, auf die über die Sprühdüsen 4 das Sterilisierungsmittel aufgeblasen wird. Zur Vermeidung von Wärmeverlusten ist die Abdeckung 5 von einer Isolierschicht 5b umgeben.

Nach unten hin wird die Abdeckung 5 von einer Bodenplatte 9 verschlossen, die eine oder eine Vielzahl von Bohrungen 9a aufweist, mit der Aufgabe, die Strömungsgeschwindigkeit des zum Verdampfen gebrachten Sterilisierungsmittels beim Eintritt in den Becher 1 zu erhöhen, um somit eine optimale Verteilung des Kondensatfilmes zu erzielen.

## Patentansprüche

1. Verfahren zum Sterilisieren von becherförmigen Behältern unter Verwendung eines Sterilisierungsmittels, das sich als Kondensatfilm an der Behälterwand niederschlägt, dadurch gekennzeichnet, daß ein Wasserstoffperoxidgemisch als Sterilisierungsmittel, durch Aufbringen auf eine Oberfläche von hoher Temperatur im becherförmigen Behälter (1) oder unmittelbar davor explosionsartig zum Verdampfen gebracht wird.

2. Verfahren nach Anspruch 1, dadurch gekennzeichnet, daß das Sterilisierungsmittel in Form eines Sprühschleiers auf ein in den Behälter (1) eingeführtes Heizelement (6) oder auf ein Heizelement (8), das in einem dem Behälter (1) vorgelagerten Raum positioniert ist, aufgebracht wird.

3. Verfahren nach Anspruch 1 oder 2, dadurch gekennzeichnet, daß die Öffnung des Behälters während des Sterilisiervorgangs verschlossen ist.

4. Vorrichtung zur Durchführung des Verfahrens nach einem der Ansprüche 1 bis 3, gekennzeichnet durch ein in den becherförmigen Behälter (1) oder vor diesen bringbares Heizelement (6 ; 8) mit einer hohen Oberflächentemperatur und durch eine auf das Heizelement (6 ; 8) gerichtete, einen Sprühschleier erzeugende Düse (4).

5. Vorrichtung nach Anspruch 4, gekennzeichnet durch eine auf die Öffnung des Behälters aufsetzbare und diesen während des Verdampfungsvorgangs verschließende Abdeckung (5) mit einem Durchlaß (5a) zum Zuführen des Sterilisierungsmittels.

6. Vorrichtung nach Anspruch 4 oder 5, dadurch gekennzeichnet, daß das in den Behälter (1) einbringbare Heizelement (6) als kegelstumpfförmige, sich zur Düse (4) in erweiternde Heizspirale ausgestaltet ist.

7. Vorrichtung nach Anspruch 4 bis 6, dadurch gekennzeichnet, daß das Heizelement (6 ; 8), die Düse (4) und die Abdeckung (5) untereinander verbunden und über ein Tragelement (3) gemeinsam zwischen einer unter und oberen Endstellung bewegbar sind.

8. Vorrichtung nach Anspruch 4 bis 7, dadurch gekennzeichnet, daß die Abdeckung (5) unter dem Heizelement (8) eine Bodenplatte (9) aufweist mit einer oder einer Vielzahl von Öffnungen (9a).

## Claims

1. Process for sterilizing pot-shaped containers, involving the use of a sterilizing agent which condenses on the container wall as a film of condensate, characterized in that a mixture containing hydrogen peroxide, serving as the sterilizing agent, is caused to undergo explosion-like evaporation as a result of being applied to a surface which is at a high temperature, either inside the pot-shaped container (1) or immediately in front of it.

2. Process according to Claim 1, characterized in that the sterilizing agent is applied either to a heating element (6) which is introduced into the container (1), or to a heating element (8) which is positioned inside a chamber located in front of the container (1), the sterilizing agent first being atomized to form a droplet-fog.

3. Process according to Claim 1 or 2, characterized in that the container mouth is closed during the sterilization operation.

4. Appliance for carrying out the process according to one of Claims 1 to 3, characterized by a heating element (6 ; 8) which has a high surface temperature and which can either be brought into the pot-shaped container (1) or be brought to a position in front of it, and further characterized by a nozzle (4) which is aimed at the heating element (6 ; 8) and which generates a fog of atomized droplets.

5. Appliance according to Claim 4, characterized by a cover (5) which possesses a passage (5a) for admitting the sterilizing agent, and which can be placed on the container mouth, closing it during the evaporation operation.

6. Appliance according to Claim 4 or 5, characterized in that the heating element (6) which can be brought into the container (1) is designed in the form of a heating spiral, exhibiting the shape of a truncated cone, its diameter increasing as the nozzle (4) is approached.

7. Appliance according to any of Claims 4 to 6, characterized in that the heating element (6 ; 8), the nozzle (4), and the cover (5) are connected, one to another, and can be moved as a group, between a lower limiting position and an upper limiting position, this movement being accomplished by means of a carrier element (3).

8. Appliance according to any of Claims 4 to 7, characterized in that the cover (5) possesses a bottom plate (9) which is located below the heating element (8) and which contains either one opening or a plurality of openings (9a).

**Revendications**

1. Procédé de stérilisation de récipients en forme de gobelet avec emploi d'un stérilisant qui se condense sous forme de film de condensat sur la paroi du récipient, caractérisé par le fait que, comme stérilisant, on fait se vaporiser de façon explosive un mélange de peroxyde d'hydrogène par application sur une surface portée à haute température dans le récipient (1) en forme de gobelet ou juste devant celui-ci.

2. Procédé selon la revendication 1, caractérisé par le fait qu'on applique le stérilisant sous la forme d'un nuage pulvérisé sur un élément chauffant (6) placé dans le récipient (1) ou sur un élément chauffant (8) qui est placé dans un espace situé devant le récipient (1).

3. Procédé selon la revendication 1 ou 2, caractérisé par le fait que l'on ferme l'ouverture du récipient pendant le processus de stérilisation.

4. Dispositif pour la mise en œuvre du procédé selon l'une des revendications 1 à 3, caractérisé par un élément chauffant (6 ; 8) à haute température de surface pouvant être placé dans le récipient (1) en forme de gobelet ou devant celui-ci, et par une buse (4) dirigée sur l'élément chauffant (6 ; 8) et produisant le nuage pulvérisé.

5. Dispositif selon la revendication 4, caractérisé par un couvercle (5) pouvant être posé sur l'ouverture du récipient, fermant celui-ci pendant le processus de vaporisation et muni d'une traversée (5a) pour l'amenée du stérilisant.

6. Dispositif selon la revendication 4 ou 5, caractérisé par le fait que l'élément chauffant (6) installé dans le récipient (1) est réalisé à la manière d'un serpentin en forme de tronc de cône allant en s'élargissant en direction de la buse (4).

7. Dispositif selon les revendications 4 à 6, caractérisé par le fait que l'élément chauffant (6 ; 8), la buse (4) et le couvercle (5) sont mutuellement raccordés et peuvent être déplacés en commun entre une position finale inférieure et une position finale supérieure au moyen d'un élément support (3).

8. Dispositif selon les revendications 4 à 7, caractérisé par le fait que le couvercle (5), en dessous de l'élément chauffant (8), comporte une plaque de fond (9) munie d'une multitude d'ouvertures (9a).

**FIG. 1**

**FIG. 2**